# EUROPEAN PATENT APPLICATION

(11) **EP 4 309 647 A1**
(43) Date of publication of application: **24.01.2024**
(21) Application number: 22186572.8
(22) Date of filing: 22.07.2022
(51) Int. Cl.: A61K 9/14, A61P 3/06, A23L 33/105, A61K 36/15, A61K 8/02

(54) **GRANULATION ACTIVE FRACTIONS OF PINE NUT SKIN, METHODS AND USES THEREOF**

(30) Priority: 19.07.2022 PT 2022118116
(71) Applicant: Universidade de Aveiro, 3810-193 Aveiro (PT)
(72) Inventor: COIMBRA RODRIGUES DA SILVA, MANUEL ANTÓNIO, 3810-083 AVEIRO (PT); MARTINS COELHO, ELISABETE VERDE, 3810-358 AVEIRO (PT); PIRES SILVA, ANA SORAIA, 6300-693 GUARDA (PT); CORETA GOMES, FILIPE MANUEL, 3810-120 AVEIRO (PT)
(74) Representative: Patentree

(57) **Abstract**

The present disclosure relates to a method for obtaining pine nut skin fractions with improved activity, comprising the steps of obtaining pine nut skin, selecting from that pine nut skin a fraction with particle size < 500 µm and a fraction with a particle size ≥ 500 µm, and their use as hypocholesterolemic agent and surfactant, respectively. Furthermore, the present invention relates to compositions comprising the fraction with particle size < 500 µm and compositions comprising the fraction with a particle size ≥ 500 µm.

## Description

### TECHNICAL FIELD

The present disclosure relates to a method for obtaining pine nut skin fractions with improved activity, and the application of such fractions in medicine, food, namely as functional food or as a nutraceutical, cosmetics, hygiene and cleaning.

### BACKGROUND

Hypercholesterolemia, or high blood cholesterol, is linked to an increased risk of cardiovascular disease, accounting for millions of deaths and a high rate of disability [1]. The decrease of LDL cholesterol (LDL-c) levels through several approaches, namely by diet control, use of statins, bile acid sequestrants, and inhibitors of cholesterol transporters in the intestine, results in a significant reduction in mortality and morbidity caused for these diseases [2]. Supplementation with plant sterols/stanols (phytosterols) reduces LDL-c levels, as early and prolonged exposure to low levels of LDL-c is associated with a low risk of developing coronary heart disease [3]. Phytosterols compete with cholesterol for solubilization in micelles, at the intestinal level. The solubilization of phytosterols in micelles prevents the solubilization and subsequent absorption of cholesterol in the intestine. Phytosterols can also co-crystallize with cholesterol, forming insoluble crystals that are excreted, as well as interfering with the hydrolysis of cholesterol in the intestinal lumen [4], [5]. Additionally, the EFSA (European Food Safety Authority) approved the health claim given to phytosterols of hypocholesterolemic agents [6]. Thus, the daily supplementation of at least 0.8 g of phytosterols favours the maintenance of low levels of LDL-c [7], which is expected to reduce the risk of coronary and cardiovascular disease [2], [3]. The use of phytosterols in the range of 1.5 to 3.0 g per day is not associated with relevant adverse health effects. However, these higher amounts may reduce carotenoid absorption and increase cardiovascular risk in patients with sitosterolemia (deficiency associated with phytosterol excretion, with an incidence of 1 in 10 million people) [8].

There are already several products enriched with plant sterols/stanols on the market, such as spreads, sauces and dairy products such as milk, yogurt and cheese. Some of the best-known functional foods are the yogurt brand Benecol^{®}, in which stanol esters are used [9], and the yoghurt and margarine from Danacol^{®} brand and margarine Becel pro.activ brand, in which sterol esters are incorporated [10], [11]. The incorporation of phytosterols in their esterified form is only related to the increase in their solubility in lipophilic environments, and not with differences in their hypocholesteroloremic effect [12]. Differences in the hypocholesterolemic dose-response relationship between sterols and stanols are reported as not relevant [13]. However, there are differences in the solubility of cholesterol in the presence of sterols when compared to stanols [14].

Pine nut and its oil, which can be extracted through pressing or with solvents or with supercritical fluids, have hypocholesterolemic properties. These properties were attributed to fatty acid residues such as 5,9,12-cis-octadecatrienoic acid (Pinolenic acid) and its derivatives, characteristic of Asian pine species such as *Pinus koraiensis, Pinus sibirica* and *Pinus cembraides,* where their abundance may exceed 10%, with linoleic acid being the most abundant fatty acid residue in this oil [15].

The pine nut skin is a thin layer located between the pine nut shell and the kernel. The pine nut skin is a by-product of the pine nut processing industry, which corresponds to 2.4% w/w of the total weight of the pine nut [16], having an annual volume estimated in 550 tonnes worldwide [17]. The pine nut skin is easily obtained during processing, with a large part being collected in the peeling phase. It has low moisture content, a relevant factor in the microbiological stability, and low density, characteristics that correspond into low drying, transport, and storage costs. The pine nut industry only sells the kernel, which represents 3% w/w of the initial raw material, the green pine cone. The pine nut skin has no use/exploitation per se, being, together with the pine cones, destined for burning, sold at 0.07-0.08 €/kg [16]. However, the aqueous extract obtained from the crushed pine nut shell has a hypocholesterolemic effect in obese mice. It is claimed that the intragastric administration of this aqueous extract obtained from the pine nut peel reduces the total amount of serum cholesterol, triglycerides, and LDL-c, increasing the cholesterol content in high-density lipoproteins (HDL-c) [15]. Although the composition of this extract was not disclosed in the patent, being an aqueous extract, it is expected that its active principles are not triglycerides rich in pinolenic acid and its derivatives, nor in sterols.

The pine nut shell of *Pinus koraiensis* also has compounds extractable in hot water and in short-chain alcohols (C1-C4) or in mixtures of the two types of solvents. These extracts are patented for cosmetic applications due to their antioxidant, whitening and anti-wrinkle properties. [18].

These facts are disclosed to illustrate the technical problem addressed by the present disclosure.

### GENERAL DESCRIPTION

The present disclosure relates to a method for obtaining pine nut skin fractions with improved activity, comprising the steps of obtaining pine nut skin, selecting from that pine nut skin a fraction with particle size <500 µm and a fraction with a particle size ≥ 500 µm, and their use as hypocholesterolemic agent and surfactant, respectively. Furthermore, the present invention relates to compositions comprising the fraction with particle size <500 µm and compositions comprising the fraction with a particle size ≥ 500 µm.

In an embodiment, the present disclosure relates to the milling of the pine nut skin, followed by particle size separation and extraction with hydrocarbon solvents. It was surprisingly found that with this process it is possible to obtain two products with different biological applications: a first product rich in sterols with an effect on reducing the bioaccessibility of cholesterol and consequent hypocholesterolemic effect, and a second product, despite the same amount of sterols, is rich in long-chain alcohols that can be used as surfactants in food, cosmetic, cleaning and hygiene formulations.

In an embodiment, the pine nut skin is milled and sieved to obtain different granulometries. The smaller particle size, rich in sterols, polyunsaturated fatty acids and terpene compounds, can be used in food ingredients with a hypocholesterolemic effect. The larger particle sizes, rich in long-chain alcohols, polyunsaturated fatty acids, sterols, and terpenic compounds, can be used as surfactant ingredients in cosmetics, cleaning, hygiene or in the food sector.

The present disclosure has potential application in the preparation of food and cosmetic ingredients based on nuts skin, such as pine nut skin.

An aspect of the present disclosure relates to a method for obtaining pine nut skin fractions with improved activity, comprising the following steps:
obtaining grinded pine nut skin,
selecting a fraction of grinded pine nut skin with a particle size <500 µm and a fraction with a particle size ≥ 500 µm.

In an embodiment, the method further comprises a step of milling the pine nut skin to obtain milled pine nut skin.

In an embodiment, the step of milling is performed with a blade grinder, roller mill, hammer mill, disc mill or ball mill; preferably a blade grinder.

In an embodiment, the step of milling is performed with a blade grinder at 20000 rpm and during 1 min.

In an embodiment, the selection step is performing by sieving.

In an embodiment, the mesh size of the sieve in the sieving step is up to 500 µm; preferably ranging from 1 µm to 500 µm; more preferably ranging from 50 µm to 500 µm.

In an embodiment, the mesh size of the sieve in the sieving step is at least 500 µm inclusive; preferably ranging from 500 µm to 1 mm; more preferably ranging from 500 µm to 700 µm.

In an embodiment, the method further comprises submitting the obtained fraction to a solid-liquid extraction in order to obtain a bioactive extract; preferably with organic solvent; more preferably a solid-liquid extraction with n-hexane as solvent.

In an embodiment, the extract obtained using particles with a particle size up to 500 µm comprises less than 10 % (w/w) of long-chain aliphatic alcohols; preferably less than 7% (w/w); more preferably 6% (w/w).

In an embodiment, the extract obtained using particles with a particle size up to 500 µm comprises:
fatty acids, in a concentration ranging from 55-70% (w/w); preferably 62.55% (w/w);
sterols, in a concentration ranging from 2.5-3.5% (w/w); preferably 3.15% (w/w); diterpenic acids, in a concentration ranging from 3.5-4.5% (w/w); preferably 3.95% (w/w); and
long-chain aliphatic alcohols, in a concentration ranging from 4.5-5.8% (w/w); preferably 5.19% (w/w).

In an embodiment, the extract obtained using particles with a particle size of at least 500 µm inclusive comprises more than 20% (w/w) of long-chain aliphatic alcohols; preferably more than 25% (w/w); more preferably 26% (w/w).

In an embodiment, the extract obtained using particles with a particle size of at least 500 µm inclusive comprises
fatty acids, in a concentration ranging from 44-54% (w/w); preferably 49.70% (w/w);
sterols, in a concentration ranging from 3.5 - 5% (w/w); preferably 4.13% (w/w); diterpenic acids, in a concentration ranging from 2-3.50 % (w/w); preferably 2.91% (w/w);
long-chain aliphatic alcohols, in a concentration ranging from 23-29% (w/w); preferably 25.91% (w/w);

Another aspect of the present disclosure relates to a composition of pine nut skin particles comprising a size of at least 500 µm, preferably ranging from 500 µm to 1000 µm, more preferably ranging from 500 µm to 700 µm, or the extract of said fraction.

Another aspect of the present disclosure relates to the use of the fraction comprising pine nut skin particles comprising a size of at least 500 µm or the extract of said fraction or the composition as a surfactant or as an emulsifying agent.

Another aspect of the present disclosure relates to a cosmetic composition comprising the fraction with pine nut skin particles comprising a size of at least 500 µm or the extract of said fraction and a cosmetic suitable carrier; preferably wherein said cosmetic composition is a lotion, a serum, a cream or a gel.

Another aspect of the present disclosure relates to a composition of pine nut skin particles comprising a size up to 500 µm, preferably ranging from 1 µm to 500 µm, more preferably ranging from 50 µm to 500 µm, or the extract of said fraction.

Another aspect of the present disclosure relates to an ingestible composition, capsule or tablet comprising the composition, or the extract previously described and a pharmaceutical suitable carrier or a food suitable carrier.

Another aspect of the present disclosure relates to a foodstuff comprising the ingestible composition, preferably wherein the food stuff is a beverage, a dairy product, a nutraceutical product, a sauce, a dessert, a spread, charcuterie, or fruit filling, preferably milk beverages, ice cream, ice milk, sherbets, yogurts, or smoothies.

Another aspect of the present disclosure relates to the fraction comprising pine nut skin particles comprising a size up to 500 µm or the extract of said fraction or the respective composition for use in medicine.

Another aspect of the present disclosure relates to the fraction comprising pine nut skin particles comprising a size up to 500 µm or the extract of said fraction or the respective composition or the foodstuff for use in the prevention or treatment of hypercholesterolemia.

Another aspect of the present disclosure relates to the use of the fraction comprising pine nut skin particles comprising a size up to least 500 µm or the extract of said fraction or the respective composition or the foodstuff in the prevention of the cholesterol level increase.

### BRIEF DESCRIPTION OF THE DRAWINGS

The following figures provide preferred embodiments for illustrating the disclosure and should not be seen as limiting the scope of the present disclosure.

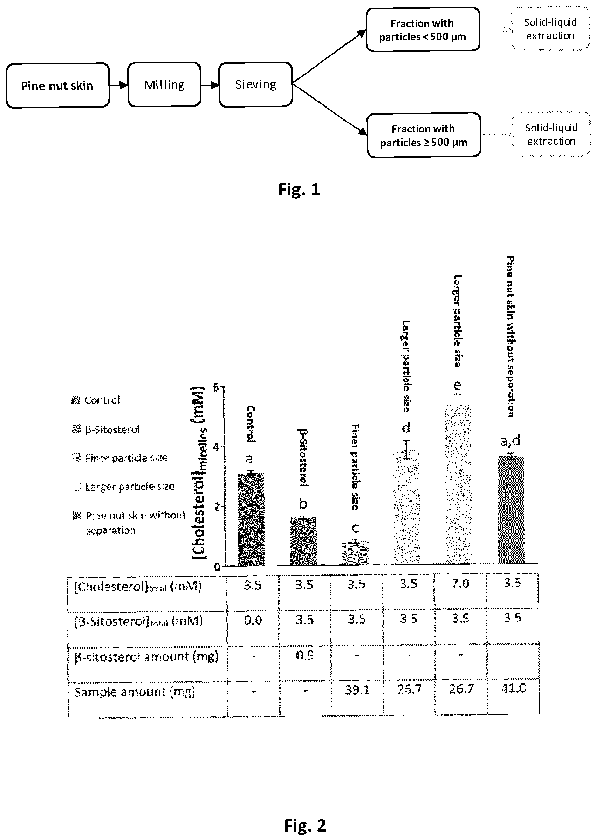

**Figure 1****:** Flowchart representing the granulometric fractionation process of the present disclosure.
**Figure 2****:** Cholesterol solubility index (mM) obtained in an intestinal model using GDCA bile salt micelles (50 mM), with the addition of β-sitosterol (blue), lipophilic extracts of finer (< 500 µm) (orange) and larger (≥500 µm) (green) particle sizes and pine nut skin without particle size separation (brown), in the equivalent amount of 3.5 mM of β-sitosterol. The lipophilic extracts' amount of the larger particle size (≥500 µm) and of the finer particle size (< 500 µm) were adjusted to correspond to an equivalent amount of 3.5 mM of β-sitosterol.

The control (gray) corresponds to the maximum solubility of cholesterol in the presence of bile salt 50 mM, corresponding to a value of 3.2 mM. The lipophilic extract of the larger particle size was tested with two cholesterol concentrations (3.5 mM and 7.0 mM). Samples with the same character (a, b, c, d, e) represent values that are not statistically different (p <0.05), after analysis of one-way ANOVA.

### DETAILED DESCRIPTION

The present disclosure relates to a method for obtaining pine nut skin fractions with improved activity, comprising the steps of obtaining pine nut skin, selecting from that pine nut skin a fraction with particle size < 500 µm and a fraction with a particle size ≥ 500 µm, and their use as hypocholesterolemic agent and surfactant, respectively. Furthermore, the present invention relates to compositions comprising the fraction with particle size < 500 µm and compositions comprising the fraction with a particle size ≥ 500 µm.

In an embodiment, the present disclosure relates to the obtention of extracts with hypocholesterolemic activity and extracts with surfactant activity, namely emulsifying activity, from an unexplored source - the pine nut skin.

In an embodiment, the present disclosure includes the process of obtaining lipophilic extracts from pine nut skin particles with two different granulometries. This disclosure comprises the analysis of the composition of lipophilic extracts and their evaluation in an *in vitro* intestinal model in order to infer their effect on cholesterol bioaccessibility.

In an embodiment, the separation of fractions by particle size allows to obtain two extracts, from the same by-product, with antagonistic effects on the solubilization of cholesterol that would otherwise be annulled. The compounds present in the finer particles, for example of size < 500 µm, decrease the solubilization of cholesterol and, consequently, its bioaccessibility, contributing to the hypocholesterolemic effect of this ingredient. By granulometric separation, it is possible to separate these compounds from the compounds present in larger particles (≥ 500 µm), which, due to their emulsifying properties, promote an increase in the solubilization of lipophilic compounds, including cholesterol. The emulsifying properties of these larger particles are due to the presence of a higher concentration of long-chain alcohols and polyunsaturated free fatty acids, sterols, and terpenic compounds. The emulsifying properties of non-protein and plant-based compounds, such as those of the extract that can be obtained from the larger particles of the pine nut skin, are requested for food, cosmetic, cleaning, and hygiene applications.

In an embodiment, the main lipophilic compounds present in the particles of pine nut skin with different granulometry are the same, differing mainly in the percentage of long chain alcohols. However, compounds that are described in the literature as promoting the decrease in cholesterol bioaccessibility, that are phytosterols, exist in similar concentrations in both extracts obtained from particles of different sizes. Thus, it is not possible to infer, without the size separation step, this very distinctive and even antagonistic behavior regarding the hypocholesterolemic activity of these two fractions.

In an embodiment, the present disclosure proposes the use of the lipophilic extract from the finer particles (< 500 µm), or the finer particles as a whole, obtained directly from the pine nut skin by simple grinding and sieving, as a food ingredient capable of decreasing the absorption of cholesterol at the intestinal level.

In an embodiment, the present disclosure proposes the use of the lipophilic extract from the larger particles (≥ 500 µm), or the larger particle as a whole (without extraction), obtained directly from the pine nut skin by simple grinding and sieving, as a food, cosmetic, cleaning and hygiene ingredient, with emulsifying ability.

In an embodiment, the present disclosure relates to the obtention of lipophilic extracts with different functions from the same by-product. It is demonstrated, for the first time, that the grinding of pine nut skin followed by particle size separation promotes the separation of particles with different chemical composition, some with hypocholesterolemic properties and others with emulsifying properties. The lipophilic compounds of the larger particles (≥ 500 µm in size), are composed of free fatty acids (50% w/w), long-chain aliphatic alcohols (26% w/w), sterols (4% w/w), and terpenic compounds (3% w/w). The smallest particles (< 500 µm in size), are composed of free (62% w/w) and esterified (8% w/w) fatty acids, terpenic compounds (5% w/w), long-chain aliphatic alcohols (6% w/w), and sterols (4% w/w).

Based on the composition of these two types of particles, it can be inferred that the total sterol content is the same (40 mg/g of extract), although different particle sizes give rise to lipophilic extracts with different activity, hypocholesterolemic or emulsifying. The effect of the extracts on cholesterol solubility was tested in an *in vitro* intestinal model, as depicted in **Figure 2** and **Table 1.** It was observed that the cholesterol solubility decreased by 74% with the lipophilic compounds from the finer particles comparing with the control, an effect superior to that observed with β-sitosterol (49% of cholesterol solubility reduction), the most representative sterol used as reference in the field of hypocholesterolemic functional foods. In the case of extracts containing lipophilic compounds from larger particles (≥ 500 µm) it was observed that when using a concentration of cholesterol of 3.5 mM, all the cholesterol was solubilized, and thus was not possible to evaluate the total extend of the capacity to increase the cholesterol solubility of this fraction. Hence, a second test was made, using a cholesterol concentration of 7.0 mM, as depicted in **Table 1.** In this case, it was surprisingly found that there was an increase in cholesterol solubility by 71%, when compared to the solubility of cholesterol in the presence of a bile acid (control sample). This increase is related to a higher concentration of long-chain alcohols (about 4 times) whose potential as a surfactant could have important applications in the field of cosmetic formulations, cleaning and hygiene, and food ingredients.

**Table 1. Effect of the extracts on cholesterol solubility (in vitro intestinal model).**

| | Control | β-Sitosterol | Finer particle size (< 500 µm (lipophilic extract) | Larger particle size (≥ 500 µm) (lipophilic extract | Larger particle size (≥ 500 µm) (lipophilic extract | Pine nut skin without particle size separation |
|---|---|---|---|---|---|---|
| [Cholesterol] total (mM) | 3.5 | 3.5 | 3.5 | 3.5 | 7.0 | 3.5 |
| [β-sitosterol] total (mM) | 0.0 | 3.5 | 3.5 | 3.5 | 3.5 | 3.5 |
| β-sitosterol amount (mg) | - | 0.9 | - | - | - | - |
| Sample amount (mg) | - | - | 39.1 | 26.7 | 26.7 | 41.0 |
| Cholesterol solubilized (mg)^{∗} | 3.1 | 1.6 | 0.8 | 3.7 | 5.3 | 3.6 |

| | | | | | | |
|---|---|---|---|---|---|---|
| ^{∗} The standard deviations obtained after solubilization of cholesterol as shown in figure 2. | | | | | | |

Thus, in an embodiment, the present disclosure relates to the obtention of pine nut skin particles with different sizes, allowing to separate by simple grinding and sieving the finer particles, able to decrease the cholesterol solubility, from the larger particles, with a relevant surfactant effect for application as ingredients for cosmetics, hygiene, cleaning, and food.

### Materials and methods

### Obtention of granulation fractions of pine nut skin

In an embodiment, 5 to 8 grams of pine nut skin of *Pinus pinea* was milled during 1 min with a blade grinder of size 6 cm at 20000 rpm and fractionated through a sieve system with size mesh of 500 µm, allowing to obtain a fraction of particles with a size equal or higher than 500 µm and another fraction of particles with a size lower than 500 µm. The particle fraction of larger size represented 15% w/w of the pine nut skin (yield of 15%), whereas the fraction of finer particle size represented 85% w/w (yield of 85%). The fractions were stored at -20 °C, under light control conditions, to avoid deterioration. The results depicted in the present disclosure pertain to these two fractions.

### Obtention of lipophilic extracts

In an embodiment, the lipophilic extracts were obtained by Soxhlet extraction using n-hexane at 80°C for 6 hours. The solvent (180 mL) was heated using a thermostatic water bath, and the sample (4 grams) was placed in a Soxhlet extractor with 60 mL. After extraction, the solvent was removed using a rotary evaporator followed by nitrogen stream, to remove all the solvent traces.

### Characterization of lipophilic extracts

In an embodiment, the lipophilic characterization of the extracts was performed by gas chromatography after conversion of the lipophilic compounds in their volatile derivatives, for example, by silylation. The compounds in free form were derivatized with 250 µL of pyridine, 250 µL of N,O-Bis(trimethylsilyl)trifluoroacetamide, and 50 µL of chlorotrimethylsilane , in the presence of 0.5 mg of tetracosane, an internal standard that allows the quantification of the lipophilic compounds in the samples.. The reaction of derivatization occured at 70 °C during 30 min. The solution containing the derivatized compounds is injected in a gas chromatograph (GC) coupled to a quadrupole mass spectrometry detector (GC-qMS). The GC was equipped with a capillary column HT5 with 30 m length, 0.25 mm internal diameter, and 0.10 µm film thickness. The samples were injected in split mode (split ratio, for example, 33), with the injector at 250 °C, and the following oven temperature program: initial temperature of 80 °C, kept during 5 min, followed by a linear increase of 4 °C/min until 260 °C, followed by a linear increase of 2 °C/min until 285 °C, which is kept for 10 minutes. The carrier gas was helium, with a flow of 35 cm/s, with the interface at 300 °C, and the source of ions at 250 °C. The mass was detected between 33 and 700 *m*/*z*, with an energy of ionization of 70 eV.

In an embodiment, in order to quantify the total lipophilic compounds (free and esterified), the compounds were hydrolyzed in alkaline medium and converted in trimethylsilyl ethers and esters, allowing their analysis as described for the analysis of the free compounds. The fraction of compounds esterified is the difference between the amount of total compounds and the amount of free compounds.

In an embodiment, the compounds were identified as trimethylsilyl derivatives by comparison of their mass spectra with the data bases and literature mass spectra [19]. For a more specific quantification, response factors can be calculated for each family of compounds in relation to the tetracosane used as internal standard. The limit of detection (LOD) and of quantification (LOQ) are usually calculated by multiplying by 3 and 10, respectively, of the ratio between the standard deviation of the signal and the slope of the calibration curve [20]. Palmitic acid (LOD 0.73 µg; LOQ 2.42 µg) was used for the quantification of saturated fatty acids and monoacylglycerides. Oleic acid (LOD 0.85 µg; LOQ 2.84 µg) was used for the quantification of monounsaturated fatty acids. Linoleic acid (LOD 0.51 µg; LOQ 1.69 µg) was used for the quantification of polyunsaturated fatty acids. Stigmasterol (LOD 0.89 µg; LOQ 2.97 µg) was used for the quantification of sterols and diterpenic acids. α-Tocopherol (LOD 0.81 µg; LOQ 2.71 µg) was used for the quantification of tocopherols. Dodecan-1-ol (LOD 0.86 µg; LOQ 2.87 µg) was used for the quantification of alcohols and alkanediols.

### Evaluation of the effect of the lipophilic extracts on cholesterol solubility

In an embodiment, to evaluate the effect of the lipophilic extracts on cholesterol solubility, an *in vitro* intestinal model with glycodehoxycholic acid (GDCA) at 37 °C and pH 7.4 was used.

In an embodiment, solutions of cholesterol labeled with carbon-13 at carbon 4 ([4-¹³C]cholesterol) with or without pine nut skin extracts was prepared in azeotropic mixtures of chloroform/methanol (87:13, v/v). Films are formed by solvent evaporation under a stream of nitrogen and heating. To remove possible traces of organic solvents, the films with cholesterol were placed on a desiccator under vacuum during, for example, 30 minutes at room temperature.

In an embodiment, the lipophilic compounds were hydrated with 600 µL of a pre-heated 50 mM GDCA in a buffered solution (pH 7.4) in deuterated water (D₂O), with a final concentration composed of 10 mM Tris-HCI, 0.15 M sodium chloride (NaCI), 1 mM ethylenediaminotetraacetic acid (EDTA), and 0.02% w/w sodium azide (NaN₃) and using, for example, trimethylsilylpropanoic acid (TSP, 5-10 mM) as internal standard. The lipophilic extracts were added to reach an amount of cholesterol equivalent to the amount of β-sitosterol (3.5 mM). The aqueous mixtures of lipophilic extracts and cholesterol were maintained in a water bath at 37 °C with continuous stirring (100 rpm) during 24 to 48 hours.

In an embodiment, the resultant emulsions were characterized by proton (¹H) and carbon (¹³C) nuclear magnetic resonance (NMR). The ¹³C RMN acquisition was performed in a spectrometer equipped with a 5 mm high field large spectrum probe with a z gradient, operating at a frequency of 500.13 MHz for ¹H NMR and at 125.8 MHz for ¹³C RMN. The ¹³C NMR spectra was obtained at 37°C, with a pulse sequence with an angle of 30°, with a spectral window of 25252 Hz, time of acquisition of 1.3 s, a relaxation time gap of 5 s, and an acquisition number of 2048 scans. The decoupling of ¹H was obtained using the WALTZ-16 decoupling sequence. The Overhauser Nuclear Effect (NOE) between ¹³C-{¹H} was obtained by the comparison of the ¹³C spectrum when completely decoupled from ¹H and ¹³C spectrum when completely decoupled from ¹H only during the acquisition period [20]. The proton spectra was obtained at 37°C with 90° pulse angle sequences with a spectral window of 7500 Hz, 1 s of acquisition time, a relaxation time delay of 5 s, and an acquisition number of 128 scans [20], [21]. The spectra was processed using MestreNova 6.1.1 software (Mestrelab Research, Santiago de Compostela, Spain).

### Results

### 1. Characterization of the lipophilic extracts

The composition of the lipophilic extracts of the two fractions of different particle size is presented in **Table 2.** The pine nut skin used as raw material was obtained from the pine species *Pinus pinea.*

In an embodiment, fatty acid residues represent 50% w/w of the lipophilic extract fraction of the particles with higher size (≥ 500 µm) and 62% w/w of the lipophilic extract fraction of the particles with lower size (< 500 µm). The fatty acid residues present in higher amount are linoleic (28% w/w and 39% w/w, respectively) and oleic acid (12% w/w and 17% w/w, respectively).

In an embodiment, five phytosterols have been found in the extracts of the two fractions. β-Sitosterol was the most abundant (30 mg/g in the high dimension particles and 32 mg/g in the lower dimension particles). In total, the phytosterols in the extract correspond to about 40 mg/g of the extract of both fractions.

In an embodiment, the γ-tocopherol occurs in similar amounts in the extracts of both fractions, with 84.4 µg/g in the fraction of higher size particles and 102.3 µg/g in the fraction of lower size particles.

In an embodiment, the diterpenic acids are also present in the extracts of both fractions, constituting 29 mg/g and 46 mg/g in the fraction with higher and lower particles size, respectively.

In an embodiment, the long chain length aliphatic alcohols are the class of compounds where the difference in the quantity between the two fractions was more notorious, accounting for 26% w/w in the extract of higher size particles (≥ 500 µm) and for 6% w/w in the extract of lower size particles (< 500 µm).

**Table 2. Free and total (the sum between free and esterified compounds are between parenthesis) compounds (mg/g extract) present in the lipophilic extracts of pine nut skin fractions**

| Compound | Higher size fraction (≥ 500 µm) (mg/g) | RSD (%) | Lower size fraction (< 500 µm) (mg/g) | RSD (%) |
|---|---|---|---|---|
| **Fatty acids** | 497.0 | | 625.5 (696.2) | |
| ***Saturated*** | 76.0 | | 100.6 (107.1) | |
| Decanoic acid (C10:0) | 0.1 | 13 | 0.2 | 13 |
| Dodecanoic acid (C12:0) | 0.1 | 9 | 0.8 | 35 |
| Myristic acid (C14:0) | 0.3 | 8 | 1.2 | 38 |
| Pentacosanoic acid (C15:0) | 0.2 | 6 | 0.5 | 32 |
| Palmitic acid (C16:0) | 25.0 | 5 | 35.3 | 10 |
| C17:0 (n-/iso-/anteiso-) | 0.4 | 1 | 0.7 | 15 |
| C17:0 (n-/iso-/anteiso-) | 0.4 | 1 | 0.5 | 14 |
| Stearic acid (C18:0) | 13.9 | 12 | 21.7 | 4 |
| Eicosanoic acid (C20:0) | 2.9 | 8 | 5.3 (6.2) | 3 |
| Behenic acid (C22:0) | 2.0 | 1 | 3.6 (3.7) | 8 |
| Tricosanoic acid (C23:0) | 0.9 | 7 | 1.5 | 15 |
| Lignoceric acid (C24:0) | 7.1 | 7 | 8.0 (9.1) | 24 |
| Pentacosanoic acid (C25:0) | 1.6 | 6 | 2.0 (2.5) | 23 |
| Cerotic acid (C26:0) | 6.6 | 15 | 10.0 | 16 |
| Heptacosanoic acid (C27:0) | 3.9 | 11 | 1.3 (3.2) | 20 |
| Montanic acid (C28:0) | 9.2 | 8 | 8.1 (9.1) | 14 |
| Triacontanoic acid (C30:0) | 1.4 | 6 | n.d. (1.1) | 1 |

| ***Unsaturated*** | 420.5 | | 523.8 (587.8) | |
|---|---|---|---|---|
| Hexadecenoic acid (C16:1) isomer | 0.3 | 5 | 0.3 (0.4) | 1 |
| Hexadecenoic (C16:1) acid isomer | 0.4 | 4 | 0.4 (0.5) | 7 |
| Octadecatrienoic acid (C18:3) | 1.1 | 14 | 1.2 | 1 |
| Otadecadienoic acid (C18:2) | 0.6 | 11 | 0.7 | 2 |
| Linoleic acid (C18:2) | 279.7 | 3 | 338.4 (388.1) | 1 |
| Oleic acid (C18:1) | 121.5 | 6 | 159.9 (173.9) | 2 |
| Octadecenoic acid (C18:1) isomer | 3.2 | 2 | 3.2 | 8 |
| Eicosatrienoic acid (C20:3) | 8.3 | 7 | 10.8 | 4 |
| Eicosadienoic acid (C20:2) | 2.6 | 1 | 5.3 | 3 |
| Eicosenoic acid (C20:1) | 2.8 | 5 | 3.6 | 4 |

| ***Diacids and esters*** | 0.5 | | 1.1 | |
|---|---|---|---|---|
| Suberic acid | 0.1 | 13 | 0.3 | 23 |
| Azelaic acid | 0.4 | 30 | 0.7 (1.0) | 19 |
| Monoacylglycerides | 5.6 | | 1.6 | |

| **Sterols** | 41.3 | | 31.5 (40.0) | |
|---|---|---|---|---|
| Campesterol | 6.4 | 6 | 3.6 (4.6) | 1 |
| Stigmasterol | 2.0 | 15 | 0.7 | 7 |
| β-Sitosterol | 30.4 | 8 | 24.3 (31.7) | 1 |
| Stigmastanol | 1.1 | 16 | 0.9 | 3 |
| Estigmast-4-en-3-one | 1.4 | 16 | 2.1 | 3 |

| **Tocopherols (µg/g)** | 84.4 | | 102.3 | |
|---|---|---|---|---|
| γ-Tocopherol | 84.4 | 11 | 102.3 | 16 |

| **Diterpenic acids** | 29.1 | | 39.5 (45.9) | |
|---|---|---|---|---|
| ***Pimaranes*** | 13.6 | | 18.0 (18.6) | |
| Pimaric acid | 7.7 | 7 | 10.3 (10.4) | 1 |
| Sandaracopimaric acid | 2.1 | 8 | 2.8 | 1 |
| Isopimaric acid | 3.8 | 7 | 5.0 (5.4) | 2 |

| ***Abietanes*** | 15.5 | | 21.4 (27.3) | |
|---|---|---|---|---|
| Abietic acid | 2.0 | 5 | 2.7 | 2 |
| Dehidroabietic acid | 7.7 | 6 | 9.2 (13.0) | 6 |
| 7β-Hydroxydehydroabietic acid | 0.7 | 5 | 1.0 (2.2) | 5 |
| 7α-Hydroxdehydroabietic acid | 3.3 | 8 | 5.5 | 3 |
| 7-Oxodehydroabietic acid | 1.9 | 8 | 3.2 (3.9) | 12 |

| **Long chain aliphatic alcohols** | 259.1 | | 51.9 (61.6) | |
|---|---|---|---|---|
| ***Primary alcohols*** | 22.4 | | 26.1 (34.3) | |
| Tricosan-1-ol | n.d. | | 0.4 (0.5) | 5 |
| Tetracosan-1-ol | 3.0 | 4 | 5.8 (7.7) | 6 |
| Pentacosan-1-ol | 1.3 | 6 | 1.8 (2.2) | 7 |
| Hexacosan-1-ol | 4.6 | 2 | 7.5 (9.8) | 7 |
| Heptacosan-1-ol | 1.2 | 6 | 1.2 (1.5) | 13 |
| Octacosan-1-ol | 7.9 | 3 | 7.6 (10.3) | 10 |
| Triacontan-1-ol | 3.8 | 8 | 1.7 (2.2) | 15 |
| Dotriacontan-1-ol | 0.5 | 8 | n.d. | |

| ***Secondary alcohols*** | 51.7 | | 8.0 (9.0) | |
|---|---|---|---|---|
| Heptacosan-10-ol | 0.5 | 9 | n.d. | |
| Nonacosan-10-ol | 51.1 | 4 | 8.0 (9.0) | 2 |

| ***Alkanediols*** | 185.0 | | 17.8 (18.3) | |
|---|---|---|---|---|
| 5,10-Heptacosanediol | 3.3 | 5 | n.d. | |
| 10,13-Nonacosanediol | 40.3 | 4 | 5.0 | 3 |
| 7,10-Nonacosanediol | 17.5 | 8 | 2.1 | 5 |
| 6,10-Nonacosanediol | 44.1 | 12 | 1.6 | 5 |
| 5,10-Nonacosanediol | 52.2 | 10 | 5.0 | 2 |
| 4,10-Nonacosanediol | 27.5 | 4 | 4.1 (4.6) | 2 |
| Total identified | 832.1 | | 750.2 (845.5) | |

| | | | | |
|---|---|---|---|---|
| RSD: relative standard deviation | | | | |

### 2. Evaluation of the effect of lipophilic extracts on the cholesterol bioaccessibility

In an embodiment, the presence of phytosterols on the extracts of both fractions indicates the potential of these extracts to affect the absorption of cholesterol at the intestinal lumen level.

In an embodiment, to evaluate this effect, the bioaccessibility of labelled cholesterol([4-¹³C]cholesterol) was measured by ¹³C NMR, using an *in vitro* intestinal model composed of glycodeoxycholic acid bile salt micelles and lipophilic extracts from pine nut skin and from the two separated fractions, added individually.

In an embodiment, the quantification of cholesterol by ¹³C NMR was possible due to the utilization of an isotopomer of cholesterol fully enriched in ¹³C in carbon 4, which has a typical peak at 44 ppm, distinguishable from GDCA resonances. The bile salt GDCA was chosen due to its prevalence in the intestinal lumen, at a concentration of 50 mM near the upper limit of what is found in the intestinal lumen (5-40 mM) [21]. This methodology allows to quantify [4-¹³C] cholesterol which is emulsified on the bile salt micelles, that is, the available quantity which is likely to be absorbed (bioaccessible). The compounds that are not emulsified due to precipitation or for having a size higher than 100 nm will not be quantified by this methodology (liquid-state NMR).

In an embodiment, as shown in **Figure 2****,** the addition of β-sitosterol in the same quantity as [4-¹³C]cholesterol (3.5 mM) results in a 49% reduction of cholesterol bioaccessability, relatively to the control (GDCA and [4-¹³C]cholesterol). The addition of lipophilic extract from finer particle size, in the quantity needed to equal β-sitosterol to 3.5 mM, resulted in a reduction of 74% of cholesterol bioaccessability, relatively to the control, allowing the emulsification of 0.8 mM of cholesterol.

On the other hand, the lipophilic extract from the larger particle size, in a concentration of β-sitosterol equal to 3.5 mM, allowed an emulsification of 5.3 mM of cholesterol, when the initial cholesterol concentration was 7.0 mM. This extract increased the emulsified cholesterol 71 % relatively to the quantity emulsified in the presence of the bile salt control.

The opposite effect observed for the two fractions, on the cholesterol solubility, is related to the presence of long chain fatty alcohols in the larger particle size. These compounds are well known due to their emulsifying properties and their amphiphilic behaviour [22]. Therefore, when testing a lipophilic extract from the whole pine nut skin, without granulometric separation (brown), all cholesterol used is solubilized (3.5 mM), showing the antagonistic effect of both fractions when not separated.

The lipophilic extract from the finer particle size (< 500 µm) showed higher hypocholesterolemic activity, leading to a decrease in the bioaccessibility of cholesterol to lower values (0.8 mM) than the ones obtained with the standard β-sitosterol (1.6 mM). Other compounds present in this extract, aside from β-sitosterol, have an impact on the cholesterol solubility. Therefore, the fraction with the finer particle size has hypocholesterolemic activity and may be used as a food ingredient.

The lipophilic extract from the larger particle size (≥ 500 µm) allowed to emulsify cholesterol in levels above the ones obtained with bile salt *per se.* Therefore, this fraction has potential as an emulsifying ingredient, for incorporation in food, cosmetic, cleaning, and hygiene applications.

### Examples

For a better comprehension of the invention, several examples of application are described, which do not intend, to limit the object range of applicability of the present invention.

Pine nut skin can be milled with the blade grinder and fractionated through a sieve system with size mesh of 500 µm, allowing to obtain a fraction of particles with a size higher than 500 µm and another fraction with a lower size than 500 µm. The particle fraction of larger size represents 15% of the pine nut skin, whereas the fraction of finer particle size represents 85%. The particles should be stored at -20 °C, under light control conditions, to avoid deterioration.

The two fractions that are obtained through sieving, can be extracted by hydrocarbons, such as *n*-hexane, using a heating system, such as a water or oil bath, using temperature above the boiling point of the solvent, using a Soxhlet apparatus, during, for example, in conditions of reflux during 6 h. After extraction, the solvent is evaporated till dryness. The yields of extraction for the larger particle size fraction is 9 %, whereas for finer particle size fraction is 8%.

In an embodiment, different grinders or mills can be employed for the milling step, namely a blade grinder, roller mill, a hammer mill, a disc mill, a ball mill or any other type of equipment that allows grinding the pine nut skin.

In an embodiment, the fractionation of pine nut skin comprises the use of a sieve system with a mesh size of 1 mm allowing to obtain particles with a size equal or higher than 1 mm and lower that 1 mm, with yields of approximately 11% and 89%, respectively.

In another embodiment, the fractionation of pine nut skin comprises the use of a sieve system with a size mesh that enable the obtention of two fractions with different particle sizes and that have different composition, namely, having different content in moisture, protein, carbohydrates, lignin, aside the differences in the quantity of secondary long chain alcohols.

In an embodiment, the extraction of lipophilic compounds is performed at a lower temperature, or even at room temperature, leading to lower yields, although with similar composition. The particle fractions with different sizes should be put in contact with the extraction solvent, for example, for 1 hour, renewing the solvent several times. The mixture should be filtered through glass fibre filter, to separate the residue from the solvent. The solvent should be evaporated till dryness.

The term "comprising" whenever used in this document is intended to indicate the presence of stated features, integers, steps, components, but not to preclude the presence or addition of one or more other features, integers, steps, components or groups thereof.

The disclosure should not be seen in any way restricted to the embodiments described and a person with ordinary skill in the art will foresee many possibilities to modifications thereof. The above described embodiments are combinable.

Where ranges are provided, the range limits are included. Furthermore, it should be understood that unless otherwise indicated or otherwise evident from the context and/or understanding of a technical expert, the values which are expressed as ranges may assume any specific value within the ranges indicated in different achievements of the invention, at one tenth of the lower limit of the interval, unless the context clearly indicates the contrary. It should also be understood that, unless otherwise indicated or otherwise evident from the context and/or understanding of a technical expert, values expressed as range may assume any sub-range within the given range, where the limits of the sub-range are expressed with the same degree of precision as the tenth of the unit of the lower limit of the range.

The following dependent claims further set out particular embodiments of the disclosure.

### References

[1] World Health Organization (WHO), "Raised Cholesterol," World Heal. Organ., vol. 17, p. 1, 2016, [Online]. Available: https://www.who.int/data/gho/indicator-metadata-registry/imr-details/3236.
[2] J. G. Robinson, B. Smith, N. Maheshwari, and H. Schrott, "Pleiotropic Effects of Statins: Benefit Beyond Cholesterol Reduction?," J. Am. Coll. Cardiol., vol. 46, no. 10, pp. 1855-1862, Nov. 2005, doi: 10.1016/j.jacc.2005.05.085.
[3] B. A. Ference et al., "Effect of long-term exposure to lower low-density lipoprotein cholesterol beginning early in life on the risk of coronary heart disease: A Mendelian randomization analysis," Ration. Pharmacother. Cardiol., vol. 9, no. 1, pp. 90-98, 2013, doi: 10.1016/J.JACC.2012.09.017.
[4] E. A. Trautwein, G. S. M. J. E. Duchateau, Y. Lin, S. M. Mel'nikov, H. O. F. Molhuizen, and F. Y. Ntanios, "Proposed mechanisms of cholesterol-lowering action of plant sterols," Eur. J. Lipid Sci. Technol., vol. 105, no. 34, pp. 171-185, 2003, doi: 10.1002/ejlt.200390033.
[5] P. M. Hunter and R. A. Hegele, "Functional foods and dietary supplements for the management of dyslipidaemia," Nat. Rev. Endocrinol., vol. 13, no. 5, pp. 278-288, 2017, doi: 10.1038/nrendo.2016.210.
[6] S. Opinion, "Scientific Opinion on the substantiation of health claims related to plant sterols and plant stanols and maintenance of normal blood cholesterol concentrations (ID 549, 550, 567, 713, 1234, 1235, 1466, 1634, 1984, 2909, 3140), and maintenance of normal pr," EFSAJ., vol. 8, no. 10, pp. 1-22, 2010, doi: 10.2903/j.efsa.2010.1813.
[7] H. Gylling et al., "Plant sterols and plant stanols in the management of dyslipidaemia and prevention of cardiovascular disease," Atherosclerosis, vol. 232, no. 2, pp. 346-360, Feb. 2014, doi: 10.1016/j.atherosclerosis.2013.11.043.
[8] A. Poli et al., "Phytosterols, Cholesterol Control, and Cardiovascular Disease," Nutrients, vol. 13, no. 8, p. 2810, Aug. 2021, doi: 10.3390/nu13082810.
[9] E. M. Vásquez-Trespalacios and J. Romero-Palacio, "Efficacy of yogurt drink with added plant stanol esters (Benecol®, Colanta) in reducing total and LDL cholesterol in subjects with moderate hypercholesterolemia: a randomized placebo-controlled crossover trial NCT01461798," Lipids Health Dis., vol. 13, no. 1, p. 125, Dec. 2014, doi: 10.1186/1476-511X-13-125.
[10] P. Lagiou, M. Løvik, R. Marchelli, A. Martin, and B. Moseley, "Danacol® and blood cholesterol Scientific substantiation of a health claim related to a low fat fermented milk product (Danacol®) enriched with plant sterols/stanols and lowering/reducing blood cholesterol and reduced risk of (coronary) heart disease purs," EFSA J., vol. 7, no. 7, pp. 1-12, 2009, doi: 10.2903/j.efsa.2009.1177.
[11] E. H. M. Temme, P. G. A. Van Hoydonck, E. G. Schouten, and H. Kesteloot, "Effects of a plant sterol-enriched spread on serum lipids and lipoproteins in mildly hypercholesterolaemic subjects," Acta Cardiol., vol. 57, no. 2, pp. 111-115, 2002, doi: 10.2143/AC.57.2.2005382.
[12] I. Demonty et al., "Continuous Dose-Response Relationship of the LDL-Cholesterol-Lowering Effect of Phytosterol Intake," J. Nutr., vol. 139, no. 2, pp. 271-284, Feb. 2009, doi: 10.3945/jn.108.095125.
[13] F. Fumeron, J. M. Bard, and J. M. Lecerf, "Interindividual variability in the cholesterol-lowering effect of supplementation with plant sterols or stanols," Nutr. Rev., vol. 75, no. 2, pp. 134-145, Feb. 2017, doi: 10.1093/nutrit/nuw059.
[14] F. M. Coreta-Gomes, W. L. C. Vaz, E. Wasielewski, C. F. G. Geraldes, and M. J. Moreno, "Quantification of Cholesterol Solubilized in Dietary Micelles: Dependence on Human Bile Salt Variability and the Presence of Dietary Food Ingredients," Langmuir, vol. 32, no. 18, pp. 4564-4574, 2016, doi: 10.1021/acs.langmuir.6b00723.
[15] L. X. B. M. S. M. H. Zhaorigetu, "Application of pine seed shell aqueous extract to hypolipidemic drugs or hypolipidemic health-care products," CN111686139A, 2020.
[16] "PineFlavour, Lda. Personal communication." .
[17] International Nut and Dried Fruit Council Foundation (INC), "Nuts and Dried Fruits Global Statistical Review." 2017, [Online]. Available: https://www.nutfruit.org/files/multimedia/1510229514_1497859419_Statistical_Yearbook_20 16-2017.pdf.
[18] C. J. W. S. J. H. C. Y. Je, "Cosmetic Composition Comprising Extract of cone scale of Pinus koraiensis Siebold et Zucc," KR20160020384A, 2016.
[19] Â. C. Salvador, M. M. Q. Simões, A. M. S. Silva, S. A. O. Santos, S. M. Rocha, and A. J. D. Silvestre, "Vine waste valorisation: Integrated approach for the prospection of bioactive lipophilic phytochemicals," Int. J. Mol. Sci., vol. 20, no. 17, 2019, doi: 10.3390/ijms20174239.
[20] F. Wang, S. Li, H. Feng, Y. Yang, B. Xiao, and D. Chen, "An enhanced sensitivity and cleanup strategy for the nontargeted screening and targeted determination of pesticides in tea using modified dispersive solid-phase extraction and cold-induced acetonitrile aqueous two-phase systems coupled with liquid chromat," Food Chem., vol. 275, pp. 530-538, Mar. 2019, doi: 10.1016/j.foodchem.2018.09.142.
[21] F. M. Coreta-Gomes et al., "In vitro hypocholesterolemic effect of coffee compounds," Nutrients, vol. 12, no. 2, pp. 1-15, 2020, doi: 10.3390/nu12020437.
[22] S. Chavda and P. Bahadur, "Micellization of a cationic gemini surfactant in aqueous solutions with different alkanols and alkanediols as additives: Effect of nonpolar chain and position of hydroxyl groups," J. Mol. Liq., vol. 161, no. 2, pp. 72-77, Jun. 2011, doi: 10.1016/j.molliq.2011.04.018.

## Claims

1. A method for obtaining pine nut skin fractions with improved activity, comprising the following steps:
obtaining grinded pine nut skin,
selecting a fraction of grinded pine nut skin with a particle size < 500 µm and a fraction with a particle size ≥ 500 µm.

2. The method according to the previous claim further comprising a step of milling the pine nut skin to obtain milled pine nut skin, preferably the step of milling is performed with a blade grinder, roller mill, hammer mill, disc mill or ball mill, preferably a blade grinder.

3. The method according to any of the previous claims wherein the selection step is performing by sieving; preferably wherein the mesh size of the sieve in the sieving step is up to 500 µm or wherein the mesh size of the sieve in the sieving step is at least 500 µm inclusive.

4. The method according to any of the previous claims further comprising submit the obtained fraction to a solid-liquid extraction in order to obtain an active extract; preferably with organic solvent; more preferably a solid-liquid extraction with n-hexane as solvent.

5. The extract obtained from the method according to any of the previous claims using particles with a size up to 500 µm comprising less than 10% (w/w) of long-chain aliphatic alcohols; preferably less than 7% (w/w); more preferably 6% (w/w); fatty acids, in a concentration ranging from 55-70% (w/w); sterols, in a concentration ranging from 2.5-3.5% (w/w); diterpenic acids, in a concentration ranging from 3.5-4.5% (w/w); and long-chain aliphatic alcohols, in a concentration ranging from 4.5-5.8% (w/w).

6. The extract obtained from the method according to any of the previous claims using particles with a size of at least 500 µm inclusive comprising more than 20 % (w/w) of long-chain aliphatic alcohols; preferably more than 25% (w/w); more preferably 26% (w/w); fatty acids, in a concentration ranging from 44-54% (w/w); sterols, in a concentration ranging from 3.5-5% (w/w); diterpenic acids, in a concentration ranging from 2-3.5% (w/w); and long-chain aliphatic alcohols, in a concentration ranging from 23-29% (w/w).

7. A composition of pine nut skin particles comprising a size of at least 500 µm, preferably ranging from 500 µm to 1000 µm, more preferably ranging from 500 µm to 700 µm, or the extract of said fraction according to the previous claim 6.

8. Use of the fraction comprising pine nut skin particles comprising a size of at least 500 µm or the extract of said fraction according to the previous claim 6 or the composition according to the previous claim 7 as a surfactant or as an emulsifying agent.

9. A cosmetic composition comprising the fraction with pine nut skin particles comprising a size of at least 500 µm or the extract of said fraction according to the previous claim 6 and a cosmetic suitable carrier; preferably wherein said cosmetic composition is a lotion, a serum, a cream or a gel.

10. A composition of pine nut skin particles comprising a size up to 500 µm, preferably ranging from 1 µm to 500 µm, more preferably ranging from 50 µm to 500 µm, or the extract of said fraction according to the previous claim 5.

11. An ingestible composition, capsule or tablet comprising the composition, or the extract described in any of the present claims and a pharmaceutical suitable carrier or a food suitable carrier.

12. A foodstuff comprising the ingestible composition described in the previous claim, preferably wherein the food stuff is a beverage, a dairy product, a nutraceutical product, a sauce, a dessert, a spread, charcuterie, or fruit filling, preferably milk beverages, ice cream, ice milk, sherbets, yogurts, or smoothies.

13. The fraction comprising pine nut skin particles comprising a size up to 500 µm or the extract of said fraction according to the previous claim 5 or the composition according to the any of the previous claims 10-11 for use in medicine.

14. The fraction comprising pine nut skin particles comprising a size up to 500 µm or the extract of said fraction according to the previous claim 5 or the composition according to the any of the previous claims 10-11 or the foodstuff according to the previous claim 12 for use in the prevention or treatment of hypercholesterolemia.

15. Use of the fraction comprising pine nut skin particles comprising a size up to 500 µm or the extract of said fraction according to the previous claim 5 or the composition according to the any of the previous claims 10-11 or the foodstuff according to the previous claim 12 in the prevention of the cholesterol level increase.
